# EUROPÄISCHE PATENTSCHRIFT

(11) **EP 0 800 522 B1**
(45) Veröffentlichungstag und Bekanntmachung des Hinweises auf die Patenterteilung: **20.02.2002**
(21) Anmeldenummer: 96945971.8
(22) Anmeldetag: 28.10.1996
(51) Int. Cl.: C07D 311/92, C07D 405/04, C07D 407/04, C07D 409/04, C08K 5/15, C09K 9/00

(54) **PHOTOCHROME DIARYL-3H-NAPHTHOPYRANE**
PHOTOCHROMIC DIARYL-3H-NAPHTHOPYRANES
DIARYL-3H-NAPHTHOPYRANNES PHOTOCHROMES

(30) Priorität: 28.10.1995 DE 19540185
(43) Veröffentlichungstag der Anmeldung: 15.10.1997
(73) Patentinhaber: Optische Werke G. Rodenstock, 80469 München (DE)
(72) Erfinder: ZINNER, Herbert, D-93080 Pentling (DE); MELZIG, Manfred, D-82234 Wessling (DE)
(74) Vertreter: Münich, Wilhelm, Dr.
(86) Internationale Anmeldenummer: DE9602049
(87) Internationale Veröffentlichungsnummer: WO9715565

(56) Entgegenhaltungen:
- EP-A- 0 629 620
- CHEMICAL ABSTRACTS, vol. 125, no. 27, 1996 Columbus, Ohio, US; abstract no. 195434z, Seite 1187; XP002029614 & JP 08 157 467 A (TOKUYAMA CORP.) 18.Juni 1996
- CHEMICAL ABSTRACTS, vol. 125, no. 27, 1996 Columbus, Ohio, US; abstract no. 221584v, Seite 1081; XP002029615 & JP 08 176 139 A (TOKUYAMA CORP.) 9.Juli 1996

## Beschreibung

### Technisches Gebiet

Die Erfindung bezieht sich auf photochrome Diaryl-3H-naphthopyrane.

### Stand der Technik

Photochrome Verbindungen besitzen die Eigenschaft, unter Einwirkung von Licht mit einem gewissen Anteil an UV-Strahlung, wie beispielsweise das Sonnenlicht oder das Licht einer Quecksilberdampflampe, ihre Farbe reversibel zu verändern. Diese Einfärbung beruht auf einer durch das energiereiche UV-Licht induzierten Bindungsöffnung im Pyranring des photochromen Naphthopyrans. Nach Entfernung der UV-Strahlungsquelle geht die Verbindung wieder in den ursprünglichen geschlossenen Zustand über.

Verschiedene Klassen von photochromen Verbindungen sind seit langem bekannt und werden in unterschiedlichen Einsatzmöglichkeiten verwendet, die eine reversible lichtabhängige Einfärbung, beispielsweise durch den UV-Anteil des Sonnenlichts, erfordern.

Verschiedene Methoden zur Einfärbung von Kunststoffgegenständen mit photochromen Verbindungen sind im Stand der Technik beschrieben. Hierzu sei nur exemplarisch auf die US-PS 4 286 957, die DE-A 35 16 568 oder die EP 0 227 337 B1 hingewiesen.

Die chemische Grundstruktur der Benzo- und Näphthopyrane ist in der US-PS 3 567 605 von Becker beschrieben. Diese Verbindungen, die bereits 1971 patentiert worden sind, haben jedoch für eine technische Anwendung bei der Herstellung photochromer Gegenstände kaum Bedeutung, da sie nur bei Temperaturen im Bereich von -40 °C eine Einfärbung in Abhängigkeit von der Bestrahlung mit ultraviolettem Licht aufweisen.

Darüber hinaus ist es aus der US-PS 5 066 818 bekannt, daß durch die Einführung mindestens einer ortho-substituierten Phenylgruppe in 3H-Stellung des Pyranrings photochrome Verbindungen erhalten werden, die zur Verwendung bei Normaltemperaturen geeignet sind. Diese Verbindungen können beispielsweise in optischen Linsen oder andere transparenten Kunststoffgegenständen eingesetzt werden.

Eine Weiterentwicklung der durch die US-PS 5 066 818 bekannten allgemeinen Naphthopyranverbindungen ist in der US-PS 5 369 158 beschrieben. Nach dieser Druckschrift weisen photochrome Naphthopyrane besonders vorteilhafte Eigenschaften auf, wenn sie in 8-Stellung des Naphthalinteils substituiert sind. Im Vergleich zu anderen Naphthopyranen mit Substituenten in 5-, 7- oder 9-Stellung weisen diese photochromen Verbindungen eine erhebliche bathochrome Verschiebung des Absorptionsmaximums sowohl im aktivierten als auch im nicht aktivierten Zustand auf. Diese Verschiebung führt im weiteren zu einer erhöhten Eindunkelungsgeschwindigkeit und einer stärkeren optische Dichte der photochromen Verbindung.

Die Naphthopyrane der US-PS 5 369 158 weisen folgende Grundstruktur auf: 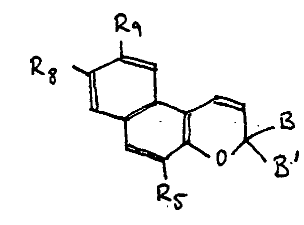 wobei R5 und R9 H, C₁-C₄-Alkoxy oder C₁-C₄-Alkyl sind, R8 Halogen, C₁-C₅-Acyloxy, Benzoyloxy, Methoxybenzoyloxy oder Di(C₁-C₅)alkylamino ist oder LO-, wobei L: C₁-C₁₂-Alkyl, C₆-C₉-Aryl(C₁-C₃)alkyl, C₅-C₇-Cycloalkyl oder mit C₁-C₄-Alkyl substituiertes C₅-C₇-Cycloalkyl ist und B und B' substituiertes oder unsubstituiertes Phenyl oder Naphthyl, Pyridyl, Thienyl, Benzothienyl, Furyl oder Benzofuryl sind, wobei der oder die Substituenten C₁-C₄-Alkyl, C₁-C₄-Halogenalkyl, C₁-C₄-Alkoxy, C₁-C₄-Alkoxy(C₁-C₄)alkyl, Di(C₁-C₅)alkylamino oder Halogen sind, unter der Voraussetzung, daß mindestens einer der Reste B oder B' substituiertes oder unsubstituiertes Phenyl ist, oder B und B' zusammen einen Adamantanrest bilden.

Ferner wird in dieser Druckschrift erwähnt, daß den photochromen Verbindungen zur Verbesserung der Stabilität und der Haltbarkeit UV-Absorber oder Stabilisatoren, wie gehinderte Aminfunktionen (HALS) oder Singulettsauerstoffauslöscher, zugesetzt werden können.

Aus der PCT/WO 95/00867 sind photochrome Naphthopyrane bekannt, die in der 9-Stellung des Naphthopyrangrundgerüsts einen Amidsubstituenten, der auch ein cyclisches Amid sein kann, aufweisen. Diese Substitution bewirkt eine Verschiebung.des Absorptionsmaximums in den längerwelligeren Bereich, führt jedoch auch zu einer verminderten maximalen Eindunkelung und einer langsameren Eindunkelungsgeschwindigkeit.

### Darstellung der Erfindung

Die Aufgabe der vorliegenden Erfindung ist die Beschreibung und Synthese von photochromen Verbindungen, die im Vergleich zu den aus dem Stand der Technik bekannten Verbindungen verbesserte Eigenschaften besitzen, wie einen guten photochromen Effekt, eine hohe Eindunkelungsgeschwindigkeit, eine große maximale Eindunkelung, eine langwellige Absorption im aktivierten Zustand, eine schnelle Aufhellungsgeschwindigkeit und eine längere Lebensdauer.

Erfindungsgemäß ist erkannt worden, daß zur Lösung dieser Aufgabe eine photochrome Verbindung geeignet ist, die eine Diaryl-3H-naphthopyranstruktur mit einer in 8-Stellung eine über ein Stickstoffatom verknüpften cyclischen Stickstoffbase aufweist, mit folgender Struktur: 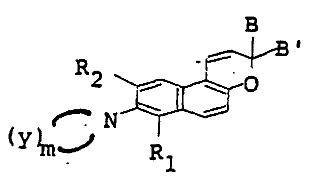 wobei
- R1 und R2:: Wasserstoff, C₁-C₄-Alkyl oder C₁-C₄-Alkoxy
- B und B':: 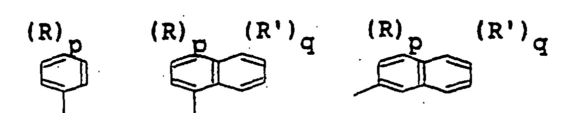 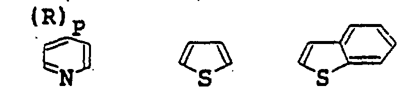 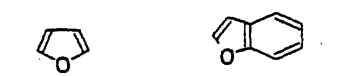 mit R und R': C₁-C₄-Alkyl, C₁-C₄-Halogenalkyl, C₁-C₄-Alkoxy, C₁-C₄-Alkoxy(C₁-C₄)-alkyl, Di(C₁-C₅)-alkylamino, Halogen, wobei Halogen = F, Cl, Br
und p, q : 0, 1, 2
- Y :: -O-, -S-, N-R'' mit R''= C₁-C₆-Alkyl, Phenyl, Benzyl
-(CZ₂)-,
wobei Z = H, C₁-C₆-Alkyl,
und zwei Z auch gemeinsam C₅-C₇-Cycloalkyl
- und m :: 3, 4, ..., 7
wobei mindestens (m-1) Reste Y -(CZ₂)- sind.

Diese Verbindungen sind den aus dem Stand der Technik bekannten Verbindungen in verschiedenen Punkten überlegen.

Die erfindungsgemäße Einführung einer cyclischen Stickstoffbase in 8-Stellung des Diaryl-3H-naphthopyrans verbessert die elektronenstabilisierende Wirkung der aus dem Stand der Technik bekannten Substituenten, wie beispielsweise -OR, durch eine stärkere Elektronendonatorwirkung der cyclischen Stickstoffbase. Diese verstärkte Stabilisierung führt überraschend nur zu einer geringen Verschiebung des Absorptionsmaximums der nicht aktivierten Form, aber zu einer starken bathochromen Verschiebung des Absorptionsmaximums der aktivierten Form der photochromen Verbindung.

Die Einführung einer cyclischen Stickstoffbase anstelle der aus dem Stand der Technik bekannten analogen Verbindungen mit offener Aminstruktur verbessert die Lebensdauer der photochromen Verbindung.

Besonders vorteilhaft ist es, gemäß Anspruch 2, eine als Hindered Amin Light Stabilizer (HALS) bekannte Verbindungsklasse in 8-Stellung der Diaryl-3H-naphthopyranstruktur einzuführen. Diese Substanzen, deren Wirkung auf dem Einfang und der Inaktivierung von Radikalen beruht, werden in polymeren Materialien als Lichtschutzmittel eingesetzt. Die üblicherweise verwendeten HALS weisen zwei wesentliche Strukturmerkmale auf. Zum einen haben sie eine freie Aminfunktion (〉N-H) und zum anderen besitzen sie in unmittelbarer Nachbarschaft zu dieser freien Aminfunktion sterisch hindernde, stark raumerfüllende Gruppen.

In neuerer Zeit sind auch polymere Substanzen vom Typ der HALS entwickelt worden. Diese weisen keine freie Aminfunktion mehr auf, sondern sind über einen Substituenten am Stickstoff polymerisiert. Jedoch besitzen sie wie die herkömmlichen HALS in ortho-Position zur Aminfunktion sterisch hindernde Gruppen. Die Lichtschutzwirkung dieser neuen Substanzen erreicht zwar nicht die Qualität der herkömmlichen HALS mit freier Aminfunktion, ist aber ausreichend für den Einsatz dieser neueren Substanzen in der in Anspruch 2 beschriebenen Ausgestaltungsform der vorliegenden Erfindung.

Die Einführung der HALS-Substanzen durch die über ein N-Atom verknüpfte cyclische Stickstoffbase in das Diaryl-3H-naphthopyrangrundgerüst erfolgt erfindungsgemäß derart, daß der Ring der cyclischen Stickstoffbase mit den Merkmalen der HALS versehen wird. Die cyclische Stickstoffbase weist also zusätzlich zu dem N-Atom, das die Verknüpfungsstelle zur Diaryl-3H-naphthopyranstruktur darstellt, eine Aminfunktion auf und in ortho-Position zu dieser Amingruppe stark raumerfüllende Substituenten, wie beispielsweise Tetramethyl, tert.-Butyl, oder andere verzweigte oder langkettige Gruppen.

Beschrieben wird der Einsatz dieser als HALS bekannten Substanzen zur Lichtstabilisierung beispielsweise in der EP 0 313 941 A1 oder in der Zeitschrift "farbe + lack", 95. Jahrgang, 10/1989, S. 715/ff. Das Taschenbuch der Kunststoff-Additive von Gächter/Müller, 2. Ausgabe, Carl Hanser Verlag beschreibt auf S. 144/ff den Einsatz dieser HALS als Zusatz zu Polymermischungen.

Aufgrund dieser Literatur ist es jedoch nur bekannt, die Substanzen den polymeren Materialien isoliert zuzusetzen. Keines der Dokumente gibt jedoch einen Hinweis diese Verbindungen in abgewandelter Form einer cyclischen Stickstoffbase in andere Moleküle, wie beispielweise photochrome Farbstoffe, einzubauen.

Die beschreibenen HALS-Verbindungen, die z.B. unter den Handelsnamen TINUVIN, CHIMASORB, CYASORB, SPINUVEX oder HOSTAVIN erhältlich sind, werden den photochromen Verbindungen üblicherweise in Konzentrationen von 0,01 bis 5 % zugesetzt, um die Lebensdauer zu erhöhen.

Durch die in Anspruch 2 genannte Einführung dieser Substanzen direkt in das Diaryl-3H-naphthopyrangerüst kann die nachträgliche Zugabe dieser Stabilisatoren entfallen. Dadurch vereinfacht sich die Anwendung der erfindungsgemäßen Verbindungen bei der Herstellung von Kunststoffgegenständen mit photochromen Eigenschaften. Ebenso verbessert sich die Lebensdauer der photochromen Verbindungen, da der Stabilisator direkt in das Molekül eingebaut wird. Unerwünschte Effekte wie Fehldosierung oder Entmischung werden somit vermieden. Damit kann sichergestellt werden, daß sich immer ein aktives HALS in der Umgebung des Farbstoffmoleküls befindet. Dies erfordert bei der üblichen Zugabe sehr hohe Konzentrationen, die teilweise im Monomer gar nicht mehr löslich nicht.

Ebenso vorteilhaft ist es, wie in Anspruch 3 beschrieben, die HALS-Verbindungen nicht unmittelbar in die cyclische Stickstoffbase einzubauen, sondern sie als Substituent direkt oder über eine Alkylbrücke -(CX₂)ₙ-, wobei X = H, C₁-C₆-Alkyl, und n = 0, 1, 2, ..., 6, mit der cyclischen Stickstoffbase zu verknüpfen.

Weiterhin ist erkannt worden, daß zur erfindungsgemäßen Lösung der Aufgabe auch Substanzen geeignet sind, die eine cyclische Stickstoffbase vom Typ der HALS-Verbindungen aufweisen, die über eine Alkylbrücke -(CX₂)ₙ-, wobei X = H, C₁-C₆-Alkyl, und n = 0, 1, 2, ..., 6, mit einem N-Atom in 8-Stellung des Diaryl-3H-naphthopyrangerüsts verknüpft ist. Die dritte Valenz des N-Atoms kann hierbei sowohl frei sein, als auch mit C₁-C₆-Alkyl substituiert sein.

Hierbei werden zwar die elektronenstabilisierenden Eigenschaften der cyclischen Stickstoffbase durch den größeren Abstand zum Diaryl-3H-naphthopyrangerüst leicht abgeschwächt. Die erfindungsgemäßen Verbindungen sind jedoch durch den gleichzeitig auftretenden Effekt der Lichtstabilisierung durch die HALS-Verbindung den Verbindungen aus dem Stand der Technik überlegen.

Ferner kann, wie in Anspruch 5 beschrieben, die cyclische Stickstoffbase noch ein ankondensiertes aromatisches oder heteroaromatisches System oder einen ankondensierten Cycloalkylring aufweisen.

Als Beispiele für die cyclischen Stickstoffbasen, die über das N-Atom in 8-Stellung in das Diaryl-3H-naphthopyrangerüst eingeführt werden, seien Azetidin, Pyrrolidin, Piperidin, Hexahydroazepin, Heptamethylimin, Morpholin, Thiomorpholin, N-Methylpiperazin, N-Phenylpiperazin, Indolin, Isoindolin, Benzoindolin, Tetrahydrochinolin, Tetrahydroisochinolin und N-Methylimidazolidin genannt.

## Patentansprüche

1. Photochrome Verbindung mit Diaryl-3H-naphthopyranstruktur, die in 8-Stellung eine über ein N-Atom verknüpfte cyclische Stickstoffbase aufweist, mit folgender Struktur: 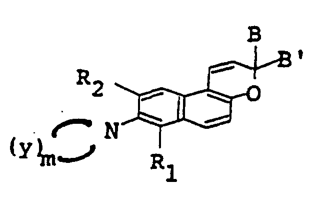 wobei
R1 und R2: Wasserstoff, C₁-C₄-Alkyl oder C₁-C₄-Alkoxy
B und B': 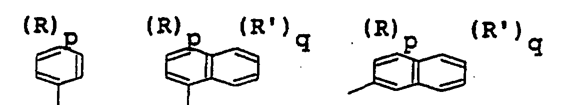 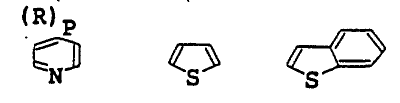 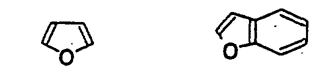
mit R und R': C₁-C₄-Alkyl, C₁-C₄-Halogenalkyl, C₁-C₄-Alkoxy, C₁-C₄-Alkoxy(C₁-C₄)-alkyl, Di(C₁-C₅)-alkylamino, Halogen, wobei Halogen = F, Cl, Br
und p, q: 0, 1, 2
Y: -O-, -S-, N-R'' mit R''= C₁-C₆-Alkyl, Phenyl, Benzyl,
-(CZ₂)-
wobei Z = H, C₁-C₆-Alkyl,
und zwei Z auch gemeinsam C₅-C₇-Cycloalkyl
und m: 3, 4, ..., 7
wobei mindestens (m-1) Reste Y -(CZ₂)- sind.

2. Photochrome Verbindung mit Diaryl-3H-naphthopyranstruktur gemäß Anspruch 1,
**dadurch gekennzeichnet, daß** die Verbindung in 8-Stellung eine über ein N-Atom verknüpfte cyclische Stickstoffbase aus der Gruppe der Hindered Amin Light Stabilizer (HALS) aufweist.

3. Photochrome Verbindung mit Diaryl-3H-naphthopyranstruktur und der in Anspruch 1 angegebenen allgemeinen Strukturformel und den dort beschriebenen Substituenten,
**dadurch gekennzeichnet, daß** der Rest R'' oder einer der Reste Z des Substituenten Y eine HALS-Verbindung oder eine über eine Alkylbrücke -(CX₂)ₙ- verknüpfte HALS-Verbindung ist,
wobei X = H, C₁-C₆-Alkyl
und n = 0, 1, 2, ..., 6.

4. Photochrome Verbindung mit Diaryl-3H-naphthopyranstruktur nach der in Anspruch 1 angegebenen allgemeinen Strukturformel und den dort angegebenen Substituenten R1, R2, B und B',
**dadurch gekennzeichnet, daß** sie in 8-Stellung ein N-Atom aufweist, das über eine Alkylbrücke -(CX₂)ₙ- mit einer HALS-Verbindung verknüpft ist,
wobei X = H, C₁-C₆-Alkyl
und n = 0, 1, 2, ..., 6
und wobei das N-Atom als weiteren Substituenten H oder C₁-C₆-Alkyl aufweist.

5. Photochrome Verbindung nach Anspruch 1,
wobei die cyclische Stickstoffbase ein ankondensiertes aromatisches oder heteroaromatisches System oder einen ankondensierten Cycloalkylring aufweist.

6. Photochrome Verbindung nach Anspruch 1,
wobei die cyclische Stickstoffbase Azetidin ist.

7. Photochrome Verbindung nach Anspruch 1,
wobei.die cyclische Stickstoffbase Pyrrolidin ist.

8. Photochrome Verbindung nach Anspruch 1,
wobei die cyclische Stickstoffbase Piperidin ist.

9. Photochrome Verbindung nach Anspruch 1,
wobei die cyclische Stickstoffbase Hexahydroazepin ist.

10. Photochrome Verbindung nach Anspruch 1,
wobei die cyclische Stickstoffbase Heptamethylimin ist.

11. Photochrome Verbindung nach Anspruch 1,
wobei die cyclische Stickstoffbase Morpholin ist.

12. Photochrome Verbindung nach Anspruch 1,
wobei die cyclische Stickstoffbase Thiomorpholin ist.

13. Photochrome Verbindung nach Anspruch 1,
wobei die cyclische Stickstoffbase N-Methylpiperazin ist.

14. Photochrome Verbindung nach Anspruch 1,
wobei die cyclische Stickstoffbase N-Phenylpiperazin ist.

15. Photochrome Verbindung nach Anspruch 5,
wobei die cyclische Stickstoffbase Indolin ist.

16. Photochrome Verbindung nach Anspruch 5,
wobei die cyclische Stickstoffbase Isoindolin ist.

17. Photochrome Verbindung nach Anspruch 5,
wobei die cyclische Stickstoffbase Benzoindolin ist.

18. Photochrome Verbindung nach Anspruch 5,
wobei die cyclische Stickstoffbase Tetrahydrochinolin ist.

19. Photochrome Verbindung nach Anspruch 5,
wobei die cyclische Stickstoffbase Tetrahydroisochinolin ist.

20. Photochrome Verbindung nach Anspruch 1,
wobei die cyclische Stickstoffbase N-Methylimidazolidin ist.

21. Photochrome Verbindung nach Anspruch 2,
wobei die cyclische Stickstoffbase 2,2,6,6-Tetramethylpiperazin ist und über das Stickstoffatom in 4-Stellung mit dem Diaryl-3H-Naphthopyran-verknüpft ist.

## Claims

1. Photochromic compound having a diaryl-3H-naphthopyrane structure, which has a cyclic nitrogen base in 8-position via a linked N atom, having the following structure : 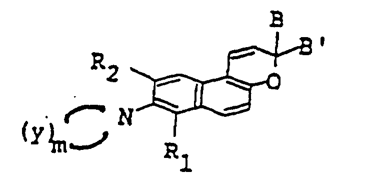
R1 and R2 being : hydrogen, C₁-C₄-alkyl or C₁-C₄-alkoxy
B and B' : 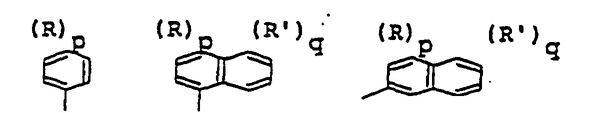 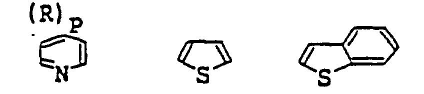 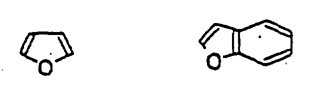
with R and R' : C₁-C₄-alkyl, C₁-C₄-halogen alkyl, C₁-C₄-alkoxy, C₁-C₄-alkoxy(C₁-C₄)-alkyl, di(C₁-C₅)-alkylamino, halogen, halogen = F, Cl, Br
and p, q : 0, 1, 2
Y : -O-, -S-, N-R" with R" = C₁-C₆-alkyl, phenyl, benzyl,
-(CZ₂)-
Z = H, C₁-C₆-alkyl,
and two Z also in common C₅-C₇-cycloalkyl
and m : 3, 4, ..., 7
at least (m-1) radicals being Y-(CZ₂)-.

2. Photochromic compound having a diaryl-3H-naphthopyrane structure according to claim 1,
**characterised in that** the compound has a cyclic nitrogen base in 8-position via a linked N atom from the group of hindered amine light stabiliser (HALS).

3. Photochromic compound having a diaryl-3H-naphthopyrane structure and the general structural formula indicated in claim 1 and the substituents described there, **characterised in that** the radical R" or one of the radicals Z of the substituent Y is a HALS compound or a HALS compound which is linked via an alkyl bridge -(CX₂)ₙ-,
X = H, C₁-C₆-alkyl
and n = 0, 1, 2, ..., 6.

4. Photochromic compound having a diaryl-3H-naphthopyrane structure according to the general structural formula indicated in claim 1 and the substituents R1, R2, B and B' given there,
**characterised in that** said compound has an N atom in 8-position which is linked via an alkyl bridge -(CX₂)ₙ- with a HALS compound,
X = H, C₁-C₆-alkyl
and n = 0, 1, 2, ..., 6,
and the N atom having H or C₁-C₆-alkyl as further substituent.

5. Photochromic compound according to claim 1, the cyclic nitrogen base having a fused aromatic or heteroaromatic system or a fused cycloalkyl ring.

6. Photochromic compound according to claim 1, the cyclic nitrogen base being azetidine.

7. Photochromic compound according to claim 1, the cyclic nitrogen base being pyrrolidine.

8. Photochromic compound according to claim 1, the cyclic nitrogen base being piperidine.

9. Photochromic compound according to claim 1, the cyclic nitrogen base being hexahydroazepine.

10. Photochromic compound according to claim 1, the cyclic nitrogen base being heptamethylimine.

11. Photochromic compound according to claim 1, the cyclic nitrogen base being morpholine.

12. Photochromic compound according to claim 1, the cyclic nitrogen base being thiomorpholine.

13. Photochromic compound according to claim 1, the cyclic nitrogen base being N-methylpiperazine.

14. Photochromic compound according to claim 1, the cyclic nitrogen base being N-phenylpiperazine.

15. Photochromic compound according to claim 5, the cyclic nitrogen base being indoline.

16. Photochromic compound according to claim 5, the cyclic nitrogen base being isoindoline.

17. Photochromic compound according to claim 5, the cyclic nitrogen base being benzoindoline.

18. Photochromic compound according to claim 5, the cyclic nitrogen base being tetrahydroquinoline.

19. Photochromic compound according to claim 5, the cyclic nitrogen base being tetrahydroisoquinoline.

20. Photochromic compound according to claim 1, the cyclic nitrogen base being N-methylimidazolidine.

21. Photochromic compound according to claim 2, the cyclic nitrogen base being 2,2,6,6-tetramethylpiperazine and being linked with the diaryl-3H-naphthopyrane via the nitrogen atom in 4-position.

## Revendications

1. Composé photochrome de structure diaryl-3H-naphthopyranne, qui présente en position 8 une base azotée cyclique liée par le biais d'un atome de N ayant la structure suivante : 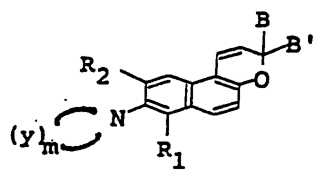 dans laquelle
R1 et R2 représentent l'hydrogène, un alkyle en C₁-C₄ ou alcoxy en C₁-C₄
B et B' représentent : 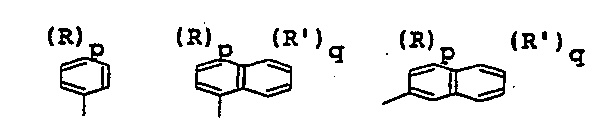 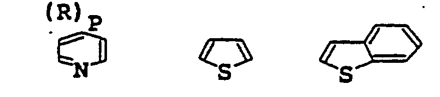 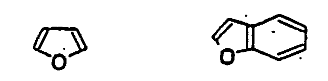
avec R et R' représentant un alkyle en C₁-C₄, un halogénure d'alkyle en C₁-C₄, un alcoxy en C₁-C₄, alcoxy(C₁-C₄)alkyle en C₁-C₄, un di(C₁-C₅)-alkylamino, un halogène, l'halogène étant = F, Cl, Br ;
et p, q représentent 0,1,2 ;
Y représente -O-, -S-, N-R", avec R" = alkyle, phényle, benzyle en C₁-C₆, -(CZ₂)-, où Z = H, alkyle en C₁-C₆, et deux Z étant tous deux cycloalkyles en C5 - C7 ;
et m représente 3, 4, ..., 7, au moins (m-1) étant un reste Y -(CZ₂)-.

2. Composé photochrome de structure diaryl-3H-naphthopyranne selon la revendication 1, **caractérisé en ce que** le composé présente en position 8 une base azotée cyclique liée par le biais d'un atome de N appartenant au groupe des stabilisateurs d'UV à amine entravée (HALS).

3. Composé photochrome de structure diaryl-3H-naphthopyranne et de formule générale indiquée dans la revendication 1 et les substituants qui y sont décrits, **caractérisé en ce que** le reste R" ou l'un des restes Z du substituant Y est un composé HALS ou un composé HALS relié par le biais d'un pont alkyle -(CX₂)ₙ-, dans lequel X = H, un alkyle en C₁-C₆ et n = 0, 1, 2, ..., 6.

4. Composé photochrome de structure diaryl-3H-naphthopyranne selon la formule générale indiquée dans la revendication 1 et les substituants qui y sont décrits R1, R2, B et B', **caractérisé en ce qu'**il présente en position 8 un atome de N relié au composé HALS par le biais d'un pont alkyle -(CX₂)ₙ-, dans lequel X = H, un alkyle en C₁-C₆ et n = 0, 1, 2, ..., 6 ; l'atome de N présentant à titre d'autres substituants un H ou un alkyle en C₁-C₆.

5. Composé photochrome selon la revendication 1, dans lequel la base azotée cyclique présente un système non condensé, aromatique ou hétéroaromatique ou un cercle cycloalkyle non condensé.

6. Composé photochrome selon la revendication 1, dans lequel la base azotée cyclique est l'azétidine.

7. Composé photochrome selon la revendication 1, dans lequel la base azotée cyclique est la pyrolidine.

8. Composé photochrome selon la revendication 1, dans lequel la base azotée cyclique est la pipéridine.

9. Composé photochrome selon la revendication 1, dans lequel la base azotée cyclique est l'hexahydroazépine.

10. Composé photochrome selon la revendication 1, dans lequel la base azotée cyclique est l'heptaméthylimine.

11. Composé photochrome selon la revendication 1, dans lequel la base azotée cyclique est la morpholine.

12. Composé photochrome selon la revendication 1, dans lequel la base azotée cyclique est la thiomorpholine.

13. Composé photochrome selon la revendication 1, dans lequel la base azotée cyclique est la N-méthylpipérazine.

14. Composé photochrome selon la revendication 1, dans lequel la base azotée cyclique est la N-phénylpipérazine.

15. Composé photochrome selon la revendication 5, dans lequel la base azotée cyclique est l'indoline.

16. Composé photochrome selon la revendication 5, dans lequel la base azotée cyclique est l'isoindoline.

17. Composé photochrome selon la revendication 5, dans lequel la base azotée cyclique est la benzoindoline.

18. Composé photochrome selon la revendication 5, dans lequel la base azotée cyclique est la tétrahydroquinoline.

19. Composé photochrome selon la revendication 5, dans lequel la base azotée cyclique est la tétrahydroisoquinoline.

20. Composé photochrome selon la revendication 1, dans lequel la base azotée cyclique est la N-méthylimidazolidine.

21. Composé photochrome selon la revendication 2, dans lequel la base azotée cyclique est la 2,2,6,6,-tétraméthylpipérazine et est liée au diaryl-3H-naphthopyranne en position 4 par le biais d'un atome d'azote.
